# EUROPÄISCHE PATENTSCHRIFT

(11) **EP 1 056 418 B1**
(45) Veröffentlichungstag und Bekanntmachung des Hinweises auf die Patenterteilung: **01.09.2004**
(21) Anmeldenummer: 98900517.8
(22) Anmeldetag: 23.01.1998
(51) Int. Cl.: A61F 2/38

(54) **KOMPONENTE FÜR EINE ENDOGELENKPROTHESE**
COMPONENT FOR AN ENDO-JOINT PROSTHESIS
ELEMENT POUR ENDOPROTHESE ARTICULAIRE

(43) Veröffentlichungstag der Anmeldung: 06.12.2000
(73) Patentinhaber: Mathys Medizinaltechnik AG, 2544 Bettlach (CH)
(72) Erfinder: GRUNDER, Beat, CH-3078 Richigen (CH); WEHRLI, Ulrich, CH-3084 Wabern (CH); MOSER, Walter, CH-3126 Kaufdorf (CH)
(74) Vertreter: Lusuardi, Werther Giovanni, Dr.
(86) Internationale Anmeldenummer: PCT/CH1998/000026
(87) Internationale Veröffentlichungsnummer: WO 1999/037251

(56) Entgegenhaltungen:
- EP-A- 0 627 203
- EP-A- 0 709 074
- US-A- 5 062 852
- US-A- 5 071 438

## Beschreibung

Die Erfindung betrifft eine Komponente für eine Endogelenkprothese gemäss dem Oberbegriff des Patentanspruchs 1.

Insbesondere bei Kniegelenkprothesen bestehen solche Komponenten aus einem metallischen, im Knochen zu verankernden Teil und einem aus Kunststoff bestehenden, eine Gleitfläche der Kniegelenkprothese enthaltenden Teil.

Aus der EP-A-0 709 074 ist eine Tibiakomponente für eine Kniegelenkprothese bekannt, welche die Merkmale des Oberbegriffs des Anspruchs 1 aufweist. Der Hauptnachteil dieser bekannten Komponente besteht jedoch darin, dass keine Spielfreiheit zwischen den beiden zusammengesetzten Teilen garantiert werden kann. Auch die aus der US-A-5 062 852 bekannte Tibiakomponenten für eine Kniegelenkprothese weist den gleichen Nachteil der fehlenden Spielfreiheit der zusammengesetzten Teilen auf.

Die Anforderungen an solche Metall/Kunststoff-Komponenten sind nämlich:
- eine einfache und sichere Handhabung unter Sterilbedingungen im Operationssaal;
- die Sicherheit gegen Desintegration der im Körper implantierten zusammengesetzten Komponente unter den auftretenden Betriebskräften, und vorallem
- die Spielfreiheit der beiden zusammengesetzten Teile.

Die Spielfreiheit ist deshalb von besonderer Bedeutung, weil Mikrobewegungen zwischen den beiden zusammengesetzten Teilen kleinste Abriebpartikel des Kunststoffteils verursachen, welche das Gewebe des Patienten belasten und zu unerwünschten biologischen Reaktionen führen können.

Weiter ist die Veränderung der mechanischen Verhältnisse in der zusammengesetzten Komponente, insbesondere in der mechanisch hoch beanspruchten Region des Kunststoffteils durch die bis heute unvermeidliche Alterung des Kunststoffen besonders kritisch. Durch die auftretende Versprödung des Kunststoffes kann die Endogelenkkomponenten ihre Funktion teilweise oder auch ganz verlieren.

Hier will die Erfindung Abhilfe schaffen. Der Erfindung liegt die Aufgabe zugrunde, eine aus einem Metall- und Kunststoffteil zusammengesetzte Komponente für eine Endogelenkprothese zu schaffen, welche auch langfristig Spielfreiheit zwischen den beiden Teilen garantiert. Durch die besondere formschlüssige Verbindung zwischen Metall- und Kunststoffteil resultiert auch bei einer Versprödung des Kunststoffes, d.h. bei einem teilweisen oder vollständigen Verlust der elastischen Eigenschaften, immer noch eine belastbare, feste Verbindung zwischen den beiden Teilen.

Die Erfindung löst die gestellte Aufgabe mit einer Komponente, welche die Merkmale des Anspruchs 1 aufweist.

Eine bevorzugte Weiterbildung besteht darin, dass der Zapfen des Kunststoffteils radiale Schlitze aufweist. Sie hat den Vorteil, dass durch entsprechende Dimensionierung und Anzahl der Schlitze die gewünschte Fügekraft eingestellt werden kann.

Eine weitere Fortbildung der Erfindung besteht darin, dass der Zapfen einen durchgehenden Hohlraum aufweist. Damit können die elastischen Eigenschaften des Zapfens, welcher nun einen Spreizstift im Hohlraum aufnehmen kann, beeinflusst werden.

Da der Fügevorgang für beide Komponenten der Endogelenkprothese im geöffneten, menschlichen Gelenk unter schwierigen Bedingungen vorgenommen werden muss, ist die gegenseitige sichere Positionierung der beiden Komponenten vor dem Fügevorgang sehr wichtig. Erfolgt die Positionierung nicht exakt, besteht die Gefahr der Beschädigung der beiden Komponenten, was zur Unbrauchbarkeit dieser Teile oder zu einem frühen Funktionsausfall des künstlichen Gelenkes führen kann. Um diese Gefahr auszuschalten, ist bei einer bevorzugten Ausführungsform der Erfindung der erste Teil mit einem Führungskamm für die Positionierung des zweiten Teils verssehen. Dieser Führungskamm unterstützt einerseits die Positionierung und verhindert anderseits bei unzureichender Positionszuordnung die Beschädigung des Zapfens. Vorzugsweise ist deshalb der Führungskamm in einem solchen Abstand von der Bohrung angeordnet, dass der Zapfen bei der Einführung in die Bohrung zu letzterer zentriert ist. Dieser Ablauf kann noch zusätzlich verbessert werden, wenn der Führungskamm eine zur Längsachse der Bohrung geneigte Keilfläche aufweist, welche mit einer am Umfang des zweiten Teils angebrachten Keilfläche zur Anlage bringbar ist. Während des Fügevorgangs kann dann die Kunststoffkomponenten entlang der schiefen Ebene des Führungskammes in ihre spielfrei verriegelte Endposition gleiten.

Die Erfindung und Weiterbildungen der Erfindung werden im folgenden anhand der teilweise schematischen Darstellungen mehrerer Ausführungsbeispiele, insbesondere am Beispiel der Tibiakomponente einer Kniegelenkprothese noch näher erläutert.

Es zeigen:
Fig. 1 einen Längsschnitt durch den ersten, metallischen Teil der erfindungsgemässen Komponente;
Fig. 2 einen Längsschnitt durch den zweiten, aus Kunststoff bestehenden Teil der erfindungsgemässen Komponente;
Fig. 3 eine Ansicht eines Spreizstiftes für das zweite Teil nach Fig. 2;
Fig. 4 einen Längsschnitt durch den zweiten Teil nach Fig. 2 mit vorläufig eingesetztem Spreizstift nach Fig. 3;
Fig. 5 einen Längsschnitt durch den auf den ersten Teil aufgesetzten zweiten Teil kurz vor Anlage am Führungskamm und Eindringen des Zapfens in die Bohrung;
Fig. 6 einen Längsschnitt durch den auf den ersten Teil aufgesetzten zweiten Teil nach erfolgtem Eindringen des Zapfens in die Bohrung und Verankerung des Wulstes im Unterschnitt;
Fig. 7 einen Längsschnitt durch die zusammengesetzte erfindungsgemässen Komponente nach Fig. 6, in welcher der Verspreizungsstift definitiv eingeführt ist;
Fig. 8 eine teilperspektivische Ansicht der Komponente nach Fig. 7;
Fig. 9 eine perspektivische Ansicht des ersten, metallischen Teils der erfindungsgemässen Komponente; sowie
Fig. 10 eine perspektivische Ansicht des zweiten, aus Kunststoff bestehenden Teils nach Fig. 2.

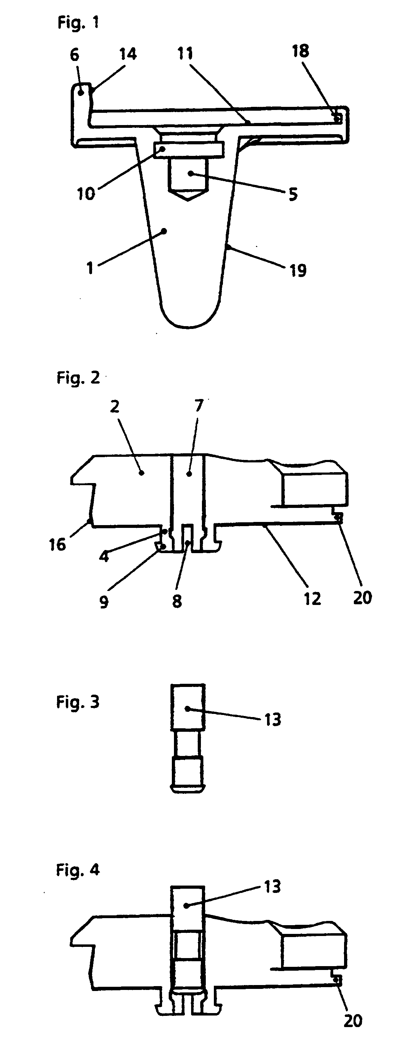

Die in den Fig. 1 und 2 schematisch dargestellte Komponente für eine Endogelenkprothese besteht im wesentlichen aus einem metallischen, im Knochen zu verankernden, ersten Teil 1 und einem aus Kunststoff bestehenden, eine Gleitfläche 3 der Endogelenkprothese enthaltenden, zweiten Teil 2.

Der erste Teil 1 weist eine Ebene 11 auf, welche - wie in Fig. 9 gezeigt, von einem peripher umlaufenden Rahmen 17 eingefasst wird, an dessen Innenseite eine Anzahl Unterschnitte 18 angebracht sind. Wie in Fig. 1 dargestellt kann dieser erste Teil 1 mittels eines Konus 19 im Knochen verankert werden.

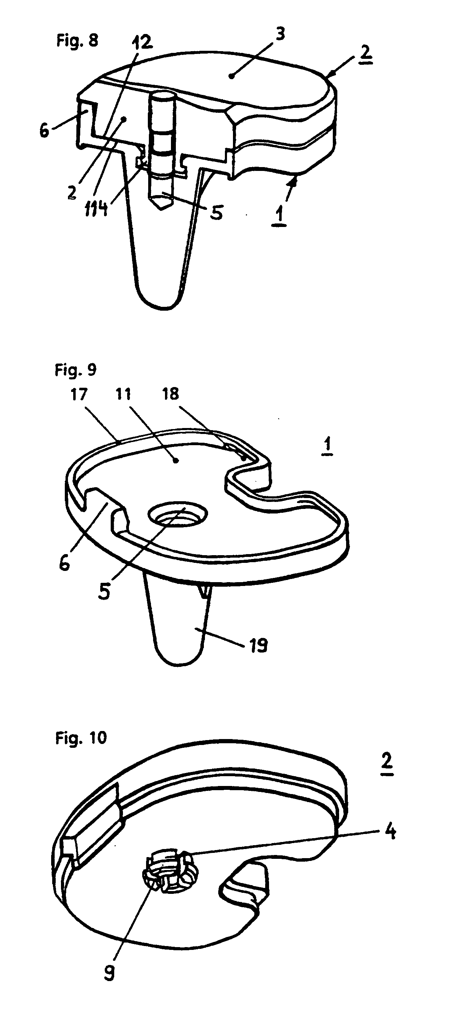

Der in Fig. 2 und 10 dargestellte zweite Teil 2 weist eine Ebene 12 auf, welche auf der Ebene 11 des ersten Teils 1 spielfrei relativ zur Ebene 11 zur Anlage bringbar ist. Zu diesem Zweck sind an äusseren Umfang des zweiten Teils 2 eine Anzahl Rippen 20 angebracht, welche in die Unterschnitte 18 des ersten Teils 1 einschnappbar sind.

Der zweite Teil 2 besitzt weitere einen senkrecht zur Ebene 12 verlaufenden Zapfen 4 mit einem peripher umlaufenden Wulst 9. Der Zapfen 4 besitzt einen durchgehenden Hohlraum 7 und weist radiale Schlitze 8 auf, so dass er radial komprimierbar ist.

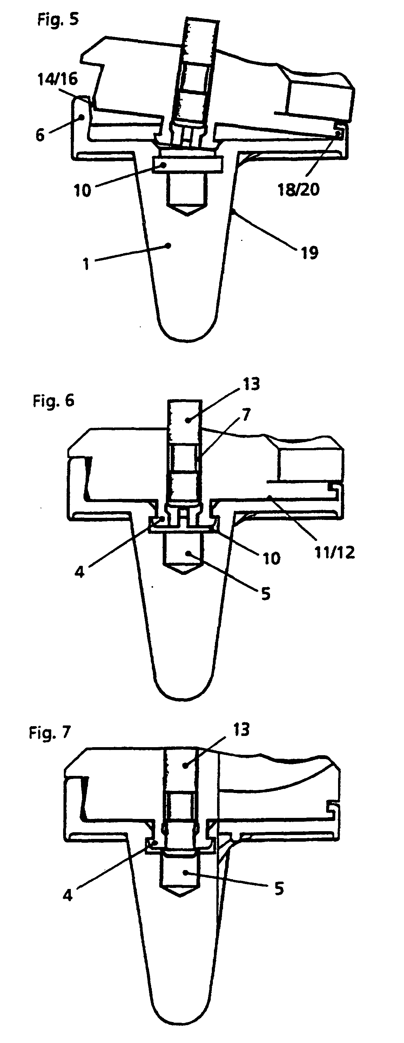

Der erste Teil 1 besitzt eine zum Zapfen komplementäre, senkrecht zur Ebene 11 verlaufende Bohrung 5 mit einem - ebenfalls komplementär zum Wulst 9 ausgebildeten - Unterschnitt 10. Bei der Einführung des Zapfens 4 in die dazu korrespondierende Bohrung 5 kann der Wulst 9 dank der Schlitze 8 komprimiert werden und weitet sich dann im Unterschnitt 10 aus, so dass der Zapfen 4 in der Bohrung 5 - wie in Fig. 6 dargestellt - blockiert ist. Der Wulst 9 und der Unterschnitt 10 sind derart angebracht, dass nach erfolgter Blockierung beide Teile 1;2 auch senkrecht zu den Ebenen 11;12 (und nicht nur in diesen Ebenen) spielfrei sind.

Wie in den Fig. 3 und 4 dargestellt, kann zur Sicherung der Blockierung des Zapfens 4 in der Bohrung 5 zusätzlich ein - vor oder nach der Montage der beiden Teile 1;2 - in den Hohlraum 7 des Zapfens 4 einführbarer Spreizstift 13 vorgesehen werden.

In den Fig. 5 - 7 ist die Montage der beiden Teile 1;2 schrittweise dargestellt. In einem ersten Schritt (Fig. 5) wird der zweite Teil 2 mit seinen Rippen 20 in den Unterschnitten 18 als Drehgelenk abgestützt und zum Führungskamm 6 hin gedreht bis die beiden Keilflächen 16 und 14 miteinander zur Anlage kommen, und der zweite Teil 2 gegenüber den ersten Teil 1 korrekt positioniert ist. Der Zapfen 4 ist dabei in einem solchen Abstand von der Bohrung 5 angeordnet, dass der Zapfen 4 bei der Einführung in die Bohrung 5 genau zentriert ist. Nach erfolgten Einschnappen des Wulstes 9 in den Unterschnitt 10 ist der Zapfen 4 in der Bohrung 5 - wie in Fig. 6 dargestellt - axial blockiert.

Durch weiteres Einschlagen des Spreizstiftes 13 (Fig. 7 und 8) in die Bohrung 7 erfolgt dann die definitive Verspreizung und Sicherung des Zapfens 4 in der Bohrung 5.

## Patentansprüche

1. Komponente für eine Endogelenkprothese, mit
A) einem metallischen, im Knochen zu verankernden, ersten Teil (1), der eine Ebene (11) aufweist und
B) einem aus Kunststoff bestehenden, eine Gleitfläche (3) der Endogelenkprothese enthaltenden, zweiten Teil (2) mit einer Ebene (12) umfasst, welche auf der Ebene (11) des ersten Teils (1) spielfrei relativ zur Ebene (11) zur Anlage bringbar ist, wobei
C) der zweite Teil (2) einen senkrecht zur Ebene (12) verlaufenden Zapfen (4) mit einem peripher umlaufenden Wulst (9) aufweist, der radial komprimierbar ist;
D) der erste Teil (1) eine senkrecht zur Ebene (11) verlaufende Bohrung (5) mit einem Unterschnitt (10) aufweist;
E) bei der Einführung des Zapfens (4) in die dazu korrespondierende Bohrung (5) der Wulst (9) komprimiert wird und sich dann im Unterschnitt (10) expandiert, so dass der Zapfen (4) in der Bohrung (5) blockiert ist;
F) Wulst (9) und Unterschnitt (10) derart positioniert sind, dass nach erfolgter Blockierung des Zapfens (4) beide Teile (1;2) auch senkrecht zu den Ebenen (11;12) spielfrei sind; und
G) der erste Teil (1) einen Führungskamm (6) für die Positionierung des zweiten Teils (2) aufweist, der in einem solchen Abstand von der Bohrung (5) angeordnet ist, dass der Zapfen (4) bei der Einführung in die Bohrung (5) zu letzterer zentriert ist;
**dadurch gekennzeichnet, dass**
H) am äusseren Umfang der Ebene (12) des zweiten Teils (2) eine Anzahl von Rippen (20) angebracht sind; und
I) auf der dem Führungskamm (6), von der Bohrng (5) aus betrachtet, gegenüberliegenden Seite des ersten Teils (1) eine Anzahl Unterschnitte (18) angebracht sind, in welche die Rippen (20) des zweiten Teils (2) als Drehgelenk abstützbar sind, um den ersten und zweiten Teil (1;2) relativ zueinander zu positionieren.

2. Komponente nach Anspruch 1, **dadurch gekennzeichnet, dass** der Zapfen (4) radiale Schlitze (8) aufweist.

3. Komponente nach Anspruch 1 oder 2, **dadurch gekennzeichnet, dass** der Zapfen (4) einen durchgehenden Hohlraum (7) aufweist.

4. Komponente nach einem der Ansprüche 1 bis 3, **dadurch gekennzeichnet, dass** zusätzlich ein in den Hohlraum (7) einführbarer Spreizstift (13) vorgesehen ist.

5. Komponente nach Anspruch 4, **dadurch gekennzeichnet, dass** der Führungskamm (6) eine zur Längsachse (15) der Bohrung (5) geneigte Keilfläche (14) aufweist, welche mit einer am Umfang des zweiten Teils (2) angebrachten Keilfläche (16) zur Anlage bringbar ist.

6. Komponente nach einem der Ansprüche 1 bis 5, **dadurch gekennzeichnet, dass** sie als Tibiakomponente einer Kniegelenkprothese ausgebildet ist.

## Claims

1. A component for an endo-joint prosthesis, with
A) a metallic first part (1) which is to be anchored in the bone and having a plane (11), and
B) a second part (2) which is made from plastic and contains a sliding surface (3) of the endo-joint prosthesis with a plane (12), which can be placed on the plane (11) of the first part (1) free of play relative to the plane (11), wherein
C) the second part (2) has a spigot (4), extending perpendicularly to the plane (12), which has a circumferentially continuous bead (9) that can be radially compressed,
D) the first part (1) has a bore (5) extending perpendicularly to the plane (11) and having an undercut (10),
E) during the introduction of the spigot (4) into the corresponding bore (5) the bead (9) is compressed and it expands into the undercut (10), so that the spigot (4) is locked in the bore (5),
F) the bead (9) and the undercut (10) are positioned in such a manner that after locking the spigot (4) both parts (1; 2) are free of play also perpendicularly to the planes (11; 12), and
G) the first part (1) is provided with a guide ridge (6) for the positioning of the second part (2), the guide ridge being arranged at such a distance from the bore (5) that the spigot (4), when being inserted into the bore (5), is centred relative to the bore, **characterised in that**
H) on the external periphery of the plane (12) of the second part (2) a plurality of robs (20) are provided, and
I) viewed from the bore (5), on that side of the first part (1 ) which is situated opposite the guide ridge (6) a plurality of undercuts (18) are provided, in which the ribs (20) of the second part (2) can rest as a hinged joint to position the first and second parts (1; 2) relative each other.

2. A component according to claim 1, **characterised in that** the spigot (4) has radial slots (8).

3. A component according to claim 1 or 2, **characterised in that** the spigot (4) has a hollow space (7) passing through it.

4. A component according to any one of claims 1 to 3, **characterised in that** additionally an expansion pin (13) is provided that can be introduced into the hollow space (7).

5. A component according to claim 4, **characterised in that** the guide ridge (6) has a wedge surface (14) that is inclined relative the longitudinal axis (15) of the bore (5), which wedge surface can be brought into contact with a wedge surface (16) provided on the circumference of the second part (2).

6. A component according to any one of claims 1 to 5, **characterised in that** it is constructed as a tibia component of a knee joint prothesis.

## Revendications

1. Composant pour une endoprothèse articulaire avec
A) une première pièce (1) métallique à ancrer dans l'os, qui présente un plan (11) et
B) une deuxième pièce (2) en matière synthétique, comportant une surface de glissement (3) de l'endoprothèse articulaire et comprenant un plan (12) que l'on peut faire adhérer au plan (11) de la première pièce (1), avec absence de jeu par rapport au plan (11),
C) la deuxième pièce (2) présentant un tourillon (4) perpendiculaire au plan (12) et muni sur sa périphérie d'un bourrelet (9) pouvant être comprimé radialement;
D) la première pièce (1) présentant un alésage (5) perpendiculaire au plan (11) et muni d'un évidement (10);
E) le bourrelet (9) étant comprimé puis se dilatant dans l'évidement (10) lors de l'introduction du tourillon (4) dans l'alésage (5) correspondant, de sorte que le tourillon (4) est bloqué dans l'alésage (5);
F) le bourrelet (9) et l'évidement (10) étant positionnés de telle sorte qu'après le blocage du tourillon (4), les deux pièces (1;2) sont également sans jeu perpendiculairement aux plans (11;12); et
G) la première pièce (1) présentant une came de guidage (6) destinée au positionnement de la deuxième pièce (2), l'écart entre cette came et l'alésage (5) étant tel que le tourillon (4) est centré par rapport à l'alésage (5) lors de son introduction dans celui-ci;
**caractérisé en ce que**
H) une quantité de nervures (20) est disposée sur la circonférence extérieure du plan (12) de la deuxième pièce (2); et **en ce que**
I) le côté de la première pièce (1) opposé à la came de guidage (6), vu depuis l'alésage (5), est muni d'une quantité d'évidements (18) sur lesquels on peut appuyer les nervures (20) de la deuxième pièce (2) en tant qu'articulation, afin de positionner la première et la deuxième pièce (1;2) l'une par rapport à l'autre.

2. Composant selon la revendication 1, **caractérisé en ce que** le tourillon (4) présente des fentes radiales (8).

3. Composant selon la revendication 1 ou 2, **caractérisé en ce que** le tourillon (4) est traversé par une cavité (7).

4. Composant selon une des revendications 1 à 3, **caractérisé en ce qu'**une cheville d'écartement (13) supplémentaire pouvant être introduite dans la cavité (7) est prévue.

5. Composant selon la revendication 4, **caractérisé en ce que** la came de guidage (6) présente une surface de blocage (14) inclinée vers l'axe longitudinal (15) de l'alésage (5) et que l'on peut faire adhérer à une surface de blocage (16) située sur le pourtour de la deuxième pièce (2).

6. Composant selon une des revendications 1 à 5, **caractérisé en ce qu'**il est conçu comme composant tibial d'une prothèse du genou.
